Europäisches Patentamt

European Patent Office

Office européen des brevets

(11) Publication number: **0 336 441 B1**

## EUROPEAN PATENT SPECIFICATION

(45) Date of publication of patent specification: **15.06.94**

(51) Int. Cl.5: **C12P  21/00**, C07K 15/00, C12N 5/00, C07K 3/18, G01N 33/577, G01N 33/86, //C12N15/00,A61K35/50, A61K37/02,C07K15/06

(21) Application number: **89106201.0**

(22) Date of filing: **07.04.89**

(54) Anti-CPBII monoclonal antibody.

(30) Priority: **08.04.88 JP 86753/88**

(43) Date of publication of application:
**11.10.89 Bulletin  89/41**

(45) Publication of the grant of the patent:
**15.06.94 Bulletin  94/24**

(84) Designated Contracting States:
**DE ES FR GB IT NL SE**

(56) References cited:

**JOURNAL OF BIOCHEMISTRY, vol. 105, 1989, pages 178-183; H. YOSHIZAKI et al.:"Isolation and characterization of an anticoagulant protein from human placenta"**

**JOURNAL OF BIOCHEMISTRY, vol. 102, no. 5, 1987, pages 1261-1273; A. IWASAKI et al.: "Structure and expression of cDNA for an inhibitor of blood coagulation isolated from human placenta: A new lipocortin-like protein"**

(73) Proprietor: **KOWA COMPANY, LTD.**
**6-29, 3-chome**
**Nishiki**
**Naka-ku Nagoya-shi Aichi-ken(JP)**

(72) Inventor: **Nagoya, Takao**
**1300-12, Naka, Tsuchiura-shi**
**Ibaraki-ken(JP)**
Inventor: **Hattori, Yukio**
**3605-905, Kashiwada-cho**
**Ushiku-shi Ibaraki-ken(JP)**

(74) Representative: **Wächtershäuser, Günter, Dr.**
**Tal 29**
**D-80331 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

BACKGROUND OF THE INVENTION

1. Field of the Invention:

This invention relates to a monoclonal antibody specific to a substance derived and purified from a human placenta and having anticoagulant activity (hereinafter abbreviated as "CPBII"), a hybridoma secreting the monoclonal antibody, and utilization of the monoclonal antibody.

2. Description of the Related Art:

The cell fusion technology has been developed rapidly since the report of Köhler and Milstein [Nature, 495-497 (1975)]. It has been known that a hybridoma obtained by fusing mammalian spleen cells and myeloma cells secretes various antibodies depending on the characteristics of the spleen cells employed. It has also been attempted to form a hybridoma, which secretes a monoclonal antibody against various biological substances such as proteins and hormones, by effecting cloning based on the characteristics of the hybridoma and also to secrete the monoclonal antibody [E. Dale Servier et al., Clinical Chemistry, 27-(11), 1797-1806 (1981)].

In the meantime, the present applicant previously succeeded in isolating and purifying a substance having anticoagulant activity (CPBII) from a human placenta and applied for a patent thereon (Japanese Patent Application No. 243778/1986). CPBII is a substance which has the following properties and is useful as a medicine.

(1) Molecular weight (SDS-polyacrylamide gel electrophoresis, reduced and non-reduced states): 73,000 ± 2,000.

(2) Isoelectric point (isoelectric column electrophoresis using an ampholyte): 6.2 - 6.6.

(3) Stability:

(a) Inactivated by a heat treatment at 50°C for 30 minutes.

(b) Stable in a pH range of 5.5 - 8.5 (at 37°C).

(c) Stable in plasma at 37°C for 15 minutes.

(4) Effects:

(a) Capable of prolonging the recalcification time.

(b) Capable of prolonging the prothrombin time.

(c) Capable of prolonging the activated partial thromboplastin time.

(5) Analysis of amino acids:

The existence of aspartic acid, threonine, serine, glutamic acid, proline, glycine, alanine, 1/2 cystine, valine, methionine, isoleucine, leucine, tyrosine, phenylalanine, histidine, lysine and arginine is recognized by the analysis of amino acids.

One example of preparation of CPBII will be described subsequently in Example 1. It may be summarized as follows.

A placenta homogenate is first prepared from the human placenta and then subjected to centrifugation. The homogenization is effected in the following manner. After cutting off the amnion and the like from the placenta, the placenta is washed thoroughly with a physiological saline, followed by homogenization by the use of a Waring blender and "Polytron". The thus-obtained homogenate was subjected to centrifugation, thereby obtaining a supernatant and sediment. The resulting placenta homogenate sediment is washed thoroughly with a buffer and is then subjected again to centrifugation to obtain a washed sediment, followed by extraction. Namely, the thus-obtained sediment of the placenta homogenate is immersed in a buffer, which contains a chelating agent such as EDTA, EGTA, oxalic acid, citric acid, sodium nitrilotriacetate or phosphoric acid, and/or another buffer containing a surfactant such as "Triton X-100", Lubrol, SDS, deoxycholic acid or the like. After allowing it to stand overnight at 4°C - 8°C, the mixture is centrifuged to collect a supernatant as an extract. Here, the extraction may be carried out by using both chelating agent and a surfactant.

The supernatant of the placenta homogenate is subjected further to ultracentrifugation at 50,000 to 100,000 x g to obtain a microsome fraction as a sediment. After washing the microsome fraction, it was extracted with a chelating agent and/or a surfactant in the same manner as described above and the resultant extract was subjected to ultracentrifugation to collect a supernatant as an extract.

The thus-obtained extract is subjected to ammonium sulfate fractionation. The ammonium sulfate fractionation is effected in the following manner. First, solid ammonium sulfate is added to 35% of its saturated concentration to the extract, followed by centrifugation to collect a supernatant. Ammonium sulfate is then added to the supernatant until its concentration reached 85% of its saturated concentration, followed by centrifugation to collect a sediment.

The resulting ammonium sulfate fraction is then purified by known isolation and purification procedures including, for example, dialysis, ion exchange chromatography, gel filtration, adsorption chromatography, hydrophobic chromatography, isoelectric point column electrophoresis, affinity chromatography using lectin or an antibody, and the like either singly or in combination, thereby obtaining CPBII. For example, a fraction obtained by subjecting the chelating agent and/or surfactant extract to ammonium sulfate fractionation is dialyzed thoroughly. The resulting dialyzate is then eluted in accordance with the linear concentration gradient method in which "DEAE-Toyopearl" is used. After an active fraction thus obtained is dialyzed, it is concentrated and subjected to gel filtration through "Sephadex G-100", thereby to obtain CPBII.

CPBII is however contained only in a trace amount in the placenta, and no sufficient specific binding is established with CPBII in various chromatographic techniques which are employed routinely. It is hence difficult to obtain CPBII in a highly pure form. Moreover, such conventional procedures require many steps and are unable to achieve any satisfactory recovery rate.

## SUMMARY OF THE INVENTION

It has therefore been desired to develop a specific purification process for obtaining high-purity CPBII easily at a high recovery rate. In order to use CPBII as an anticoagulant, it has also been desired to elucidate the mechanism of the action of CPBII and further to develop a high-sensitivity assay for CPBII as a method for measuring its blood level.

The present inventors have carried out an extensive investigation with a view toward solving these problems. As a result, it has been succeeded to obtain by a cell fusion technique a hybridoma secreting a monoclonal antibody specific to CPBII. It has also been found that the monoclonal antibody specific to CPBII can be obtained using the hybridoma and the use of the monoclonal antibody permits high-level purification and immunoassay of CPBII, thereby leading to completion of this invention.

The present invention therefore provides a monoclonal antibody specific to CPBII, a hybridoma secreting the monoclonal antibody, a purification process of CPBII featuring the use of the monoclonal antibody as an immunoadsorbent, as well as an immunoassay of CPBII featuring the use of monoclonal antibodies.

The hybridoma of this invention secretes a monoclonal antibody specific to CPBII. Use of the monoclonal antibody then makes it possible to simplify the isolation and purification process of CPBII. Moreover, the solid carrier with the monoclonal antibody couple thereon is usable repeatedly provided that it is washed. The antibody-coupled carrier is extremely useful from the industrial viewpoint, since its use can simplify the purification step and moreover, is economical.

Further, the anti-CPBII monoclonal antibody can also be used for the immunoassay of CPBII in the treatment of abnormality or a disease in the coagulation fibrinolysis system of blood.

## BRIEF DESCRIPTION OF THE DRAWING

The above and other objects, features and advantages of the present invention will become apparent from the following description and the appended claims, taken in conjuction with the accompanying drawing, in which:

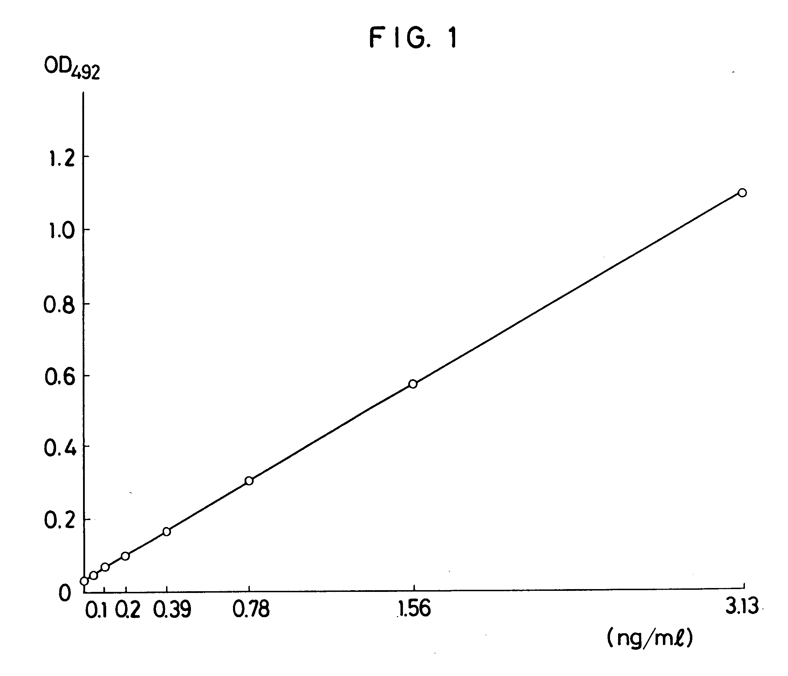

FIGURE 1 illustrates the relation between the CPBII concentration and the absorbance (at 492 nm) in the assay of CPBII in Example 4, in which CPBII-A29 was used as a coating monoclonal antibody while CPBII-A511 was used as a monoclonal antibody to be conjugated.

## DETAILED DESCRIPTION OF THE INVENTION AND PREFERRED EMBODIMENTS

The hybridoma secreting the monoclonal antibody specific to CPBII can be formed, for example, in the following manner. (1) Antibody-secreting cells are prepared from an animal which has been immunized using CPBII as an antigen. (2) Myeloma cells are prepared separately. (3) These cells are caused to fuse together. (4) The resulting hybridomas are allowed to grow selectively. (5) Antibody-secreting hybridomas are screened out from the hybridomas. (6) The intended hybridoma secreting the monoclonal antibody is obtained by cloning. Each of the above steps will next be described.

3

(1) Preparation of antibody-secreting cells:

The preparation of antibody-secreting cells may be conducted in accordance with a method known commonly in the art. This may be done by immunizing an animal with CPBII as an antigen and then collecting antibody-secreting cells from the animal. A mouse, rat, rabbit, guinea pig, sheep or the like may be mentioned by way of example as the animal. As the antibody-secreting cells, may be used those isolated from the spleen, lymph node, peripheral blood or the like. The immunization may be performed by a method in which the Freund's complete adjuvant is used in combination.

(2) Preparation of myeloma cells:

Cell strains of many mammals may be used as myeloma cells in the cell fusion. It is however preferable to use the cell strains of an animal which belongs to the same family as the animal used for the preparation of the antibody-secreting cells. It is preferable to use a cell strain having specific chemical resistance, so that unfused cells and fused cells can be separated from each other after the cell fusion by incubating both unfused and fused cells in such a way that unfused myeloma cells cannot survive but hybridomas alone are allowed to grow in a selective medium. For example, 8-azaguanine resistant cells are favorably employed because they cannot grow in HAT medium. As specific cell strains useful in the practice of this invention, may be mentioned mouse myeloma cell strains PAI, P3-X63-Ag8, P3-X63-Ag8-Ul, P3-NSl/l-Ag4-l, X63-Ag8-6.5.3., SP2/0-Agl4, FO, S194/5XXO.BU.l, MPCll-45.6.TG.1.7, etc.

(3) Cell fusion:

The cell fusion is effected usually by mixing myeloma cells and antibody-secreting cells (at a mixing ratio of 1:4 - 1:10, in general) in a medium such as MEM medium, PRMl1640 medium or IMDM medium. As a fusion promoter, may be used polyethylene glycol (PEG) the average molecular weight of which ranges from 1,000 to 6,000. PEG may be employed usually in an amount of 30 - 50%. There is also a cell fusion technique using a recently-developed electric cell fusion apparatus which relies upon high-voltage pulses. This technique can provide a superior fusion efficiency to the cell fusion using PEG provided that fusing conditions are modified a little in accordance with the kind of cells.

(4) Selective growth of hybridomas:

Cells which have gone through the cell fusion are diluted suitably with IMDM medium containing 10% FCS or a like medium, followed by centrifugation. The resultant sediment is suspended in a selective medium (for example, HAT medium) and inoculated on a 96-well microtiter plate. It is then cultured in a 5% carbon dioxide culture apparatus. Cells which have grown in the selective medium are hybridomas.

(5) Screening of antibody-secreting hybridomas:

The screening of antibody-secreting hybridomas may be effected by a method known per se in the art. No particular limitation is imposed thereon. For example, the screening may be conducted by collecting a culture with hybridomas grown therein, reacting with CPBII and then reacting the resultant product further with a second antibody labelled with an enzyme, fluorescent substance or light-emitting substance, etc.

(6) Cloning:

Cells in a culture well, which has been found to contain antibody-secreting hybridomas, are subjected to cloning in accordance with the limiting-dilution method or the like, whereby a hybridoma secreting the monoclonal antibody is obtained.

Following the above-described procedures, were obtained hybridomas secreting a monoclonal antibody specific to CPBII, i.e., hybridomas CPB-II H29, CPB-II H76, CPB-II H311 and CPB-II H511. These hybridomas are novel cells which can each secrete a monoclonal antibody specific to CPBII. These cells have hence been deposited respectively under BP-1753 (CPB-II H29), BP-1754 (CPB-II H76), BP-1755 (CPB-II H311) and BP-1756 (CPB-II H511) with Fermentation Research Institute, Agency of Industrial Science and Technology, Government of Japan.

The monoclonal antibody specific to CPBII can be formed by using the antibody-secreting hybridoma obtained above. Namely, the monoclonal antibody of this invention can be obtained from a culture

4

supernatant by culturing the antibody-secreting hybridoma in a suitable medium. In order to form the monoclonal antibody in a large volume, a mineral oil such as pristane (2,6,10,14-tetramethylpentadecane) is peritoneally administered to an animal of the same family as an animal used to obtain the myeloma cells and the antibody-secreting hybridoma is then inoculated, whereby the antibody-secreting hybridoma is allowed to grow in vivo to a large volume. According to the above-described method, the monoclonal antibody is formed at a high concentration in both serum and ascitic fluid of the inoculated animal. The separation and purification of the monoclonal antibody may be practised in accordance with a method which is used routinely for the purification of an antibody from serum.

The thus-obtained monoclonal antibody of this invention includes four types of monoclonal antibodies depending on the type of the antibody-secreting hybridoma employed, namely, CPBII-A29, CPBII-A76, CPBII-A311 and CPBII-A511. These monoclonal antibodies have characteristics shown in Table 1, in which the molecular weights were measured by SDS-polyacrylamide gel electrophoresis, the isoelectric points by isoelectric point electrophoresis (an LKB-column isoelectric point electrophoretic apparatus), and the immunoglobulin subclasses by the Ouchterlony's double immunodiffusion test [a rabbit polyclonal antibody (product of Miles Laboratory) was used].

Table 1

| Hybridoma | Monoclonal antibody | Molecular weight | Globulin class | | Isoelectric point |
|---|---|---|---|---|---|
| | | | H Chain | L Chain | |
| CPBII H29 | CPBII-A29 | 170,000 ± 5,000 | G1 | κ | 6.8 - 6.9 |
| CPBII H76 | CPBII-A76 | 160,000 ± 5,000 | G1 | κ | 6.4 - 6.5 |
| CPBII H311 | CPBII-A311 | 160,000 ± 5,000 | G1 | κ | 6.0 - 6.2 |
| CPBII H511 | CPBII-A511 | 155,000 ± 5,000 | G1 | κ | 5.6 - 5.8 |

As described above, CPBII is purified by using the monoclonal antibody of this invention as an immunoadsorbent. This may be practised, for example, by coupling the monoclonal antibody of this invention with a solid carrier such as dextran gel, agarose gel or polyvinyl gel and then subjecting crude CPBII to chromatography on a column of the monoclonal antibody coupled carrier as an immunoadsorbent. The coupling of the solid carrier and monoclonal antibody is effected in accordance with the cyanogen bromide method or via epoxy, amino, carboxyl or formyl groups.

The crude CPBII is charged into the column in which the solid carrier with the monoclonal antibody coupled thereon is packed. By eluting CPBII adsorbed on the column, CPBII can be obtained in a highly pure form.

The immunoassay of CPBII, which makes use of a conjugate of the monoclonal antibody of this invention, may be practised, for example, in the following manner. The monoclonal antibody of this invention is labelled with a labelling agent such as an enzyme, isotope or fluorescent substance. A CPBII-containing sample is then added to the resultant conjugate. The degree of labelling of the immunoreaction product between the CPBII and conjugate is thereafter measured. The ELISA method may also be used as a general method.

This invention will hereinafter be described by the following Examples.

Example 1:

Formation of hybridoma secreting anti-CPBII monoclonal antibody:

(1) Purification of antigen (CPBII):

(i) Five human placentae (about 2,500 g) were minced subsequent to removal of membranes and the like and thorough washing with a physiological saline. The thus-minced placentae were added with 2 ℓ of a 50 mM tris-hydrochloric acid buffer (pH 7.4) and then ground in a Waring blender, followed by further comminution in "Polytron". The resulting homogenate was subjected to centrifugal separation at 7,000 r.p.m. for 15 minutes to collect a sediment. Two liters of the 50 mM tris-hydrochloric acid buffer (pH 7.4) were added again to the thus-collected sediment, and the resulting mixture was homogenized in "Polytron" and then subjected to centrifugal separation at 7,000 r.p.m. for 15 minutes to obtain a washed sediment. The above procedure was repeated several times until blood components were removed to obtain about 900 g of a washed sediment finally.

(ii) About 2 liters of a 50 mM tris-hydrochloric acid buffer (pH 7.4) containing 50 mM of EDTA were added to 900 g of the sediment obtained in the above procedure (i), followed by homogenization in the Waring blender. The resulting homogenate was agitated overnight at 4°C, followed by centrifugal separation at 7,000 r.p.m. for 15 minutes to obtain 2 liters of an extract.

(iii) Solid ammonium sulfate was added to the extract obtained in the above (ii) to 35% of its saturated concentration. After allowing the resultant mixture to stand at 4°C for 30 minutes to several hours, it was centrifuged at 7,000 r.p.m. for 15 minutes to collect a supernatant. Ammonium sulfate was added further to the supernatant to 85% of its saturated concentration. The resultant mixture was allowed to stand at 4°C for 2 hours, followed by centrifugation at 7,000 r.p.m. for 15 minutes to collect a sediment. The thus-obtained sediment was dissolved in a small amount of a 20 mM tris-hydrochloric acid buffer and thoroughly dialyzed overnight at 4°C against the same buffer. The precipitate formed during the dialysis was removed by centrifugation at 7,000 r.p.m. for 15 minutes to obtain 390 mℓ of a dialyzate.

(iv) The thus-obtained dialyzate was adsorbed on DEAE-Toyopearl (φ 5.5 x 19 cm) which had been equilibrated with a 20 mM tris-hydrochloric acid buffer (pH 7.4) and washed thoroughly with the same buffer. Using 4-liter portions of the same buffer which portions contained 0 to 0.3 M of sodium chloride respectively, elution was then performed at a rate of 20 mℓ per fraction in accordance with the linear concentration gradient method. Active fractions were eluted around a sodium chloride concentration of approximately 0.2 M, thereby obtaining 200 mℓ of active fractions.

(v) The resultant active factions were concentrated through a "DIAFLOW Membrane Filter YM-10". The concentrate was subjected to gel filtration using "Sephadex G-100" (φ 4.5 x 75 cm) and eluted at a rate of 8 mℓ per fraction with a physiological saline. Active fractions 70 - 82 were collected and concentrated by ultrafiltration to obtain 14 mℓ of CPBII (protein weight: 59.3 mg, Lowry method).

(2) Preparation of immunized spleen cells:

The above-purified CPBII (100 μg) was emulsified in the Freund's complete adjuvant and administered intraperitoneally to BALB/C mice.

CPBII (50 μg/administration) and an adjuvant emulsion were thereafter administered twice at an interval of 2 weeks and finally, 50 μg of CPBII was administered solely to complete the immunization.

Three days later, the mouse was sacrificed. After taking out the spleen and chopping same, it was filtered through a 100-mesh nylon mesh to obtain isolated spleen cells.

(3) Preparation of hybridoma:

A hypotonic solution (155 mM ammonium chloride) was added to the thus-obtained immunized spleen cells to subject red blood cells to hemolysis. The cells were then washed with Iscove's modified Dulbecco's medium (IMDM). On the other hand, mouse myeloma cells PAI were also washed twice with IMDM. Both cells were counted. The spleen cells and PAI cells were combined together at a ratio of 5:1, followed by centrifugation. The supernatant was decanted out. After adding a fusing buffer (0.25 M mannitol, 0.1 mM CaCl$_2$, 0.1 mM MgCl$_2$, 0.2 mM tris-hydrochloric acid; pH 7.2) to the resultant cell sediment, the mixture thus formed was thoroughly stirred and then centrifuged. That procedure was repeated twice. The fusing buffer was added to the resultant cell sediment to adjust the cell population to $4 \times 10^7$/m$\ell$. A 100-200 $\mu\ell$ portion of the cell mixture was placed dropwise between electrodes of a cell fusion apparatus ("SSH-I Model", trade name; manufactured by Shimadzu Corp.). After applying a voltage of 40 V at 1 MHz for 10 seconds across the electrodes, an electric pulse was impressed several times at 300 V for 1/60 second. The cell mixture was allowed to stand for 5 minutes. Thereafter, the cells between the electrodes were washed out with IMDM and transferred into a centrifugal tube. The centrifugal tube was centrifuged at 1,000 rpm for 8 minutes.

The resulting sediment was suspended in IMDM which had been added with 10% of fetal calf serum (FCS). The suspension was centrifuged again and the resultant supernatant was decanted out.

The thus-obtained sediment was suspended again in (HAT-)10% FCS-added IMDM in which $10^{-4}$ M of hypoxanthine, $4 \times 10^{-7}$ M of aminopterin and $1.6 \times 10^{-5}$ M of thymidine had been added in advance, so that a cell population of $4 \times 10^6$/m$\ell$ was given. The resultant suspension was poured in 100-$\mu\ell$ portions into the individual wells of a 96-well microtiter plate. Each well was added with 50 $\mu\ell$ of the medium every third - fourth day. Growth of cells was observed.

It was confirmed that hybridomas were only allowed to grow owing to the selective action of HAT.

(4) Screening of antibody-secreting hybridomas:

The culture in a well, in which hybridomas had grown, was collected and a test was performed by enzyme immunoassay to determine if antibody-secreting hybridomas secreting an antibody to CPBII were contained there. First of all, CPBII was poured at a rate of 0.1 $\mu$g/100$\mu\ell$/well into each well of a 96-well microtiter plate ("Immunoplate I", product of NUNC Company). The microtiter plate was left over at 25°C for 18 hours so as to adsorb CPBII. Thereafter, a culture as a sample was poured at a rate of 100 $\mu\ell$/well to react at 25°C for 2 hours. After washing the culture three times with a phosphate-buffered saline containing 0.05% of "Tween 20" (PBS-Tween), horse radish peroxidase conjugated goat anti-mouse IgG (product of KPL Laboratories, Inc.) was added at a rate of 100 $\mu\ell$/well and two hours later, the culture was washed three times with PBS-Tween. Each well was then added with a 0.1 M citric acid-phosphate buffer (pH 5.0) containing 0.01% of hydrogen peroxide solution and 0.4 mg/m$\ell$ of orthophenylene diamine (product of Sigma Chemical Company) and the absorbance of the culture in each well was measured at a wavelength of 492 nm.

Since color development was observed only in wells where an antibody to CPBII existed in the sample, cells were collected from the wells which were stained.

(5) Cloning of hybridomas secreting a monoclonal antibody specific to CPBII:

Abdominal cells collected by injecting IMDM into the abdominal cavity of a mouse were used as feeder cells.

The abdominal cells suspended at $1 \times 10^5$ cells/m$\ell$ in 10% FCS-added IMDM were poured in 100-$\mu\ell$ portions into the individual wells of a 96-well microtiter plate. On the following day, antibody-secreting hybridomas were prepared at a concentration of 5 cells/m$\ell$ and poured in 100-$\mu\ell$ portions into the individual wells. Every third day, the culture medium was replaced by a fresh supply of the same medium, and culture supernatants were successively sampled out from wells in which hybridomas had grown to an appropriate volume. Confirmation of the secretion of the antibody was conducted by the same method as that described above. The cultures of positive wells were cloned again to obtain hybridomas secreting an anti-CPBII monoclonal antibody. Four types of hybridomas were obtained. As already shown in Table 1, they were named CPB-II H29, CPB-II H76, CPB-II H311 and CPB-II H511 in accordance with the types of the anti-CPBII monoclonal antibodies which they secrete respectively.

Example 2:

Preparation of anti-CPBII monoclonal antibody:

Seven-weeks-old BALB/C mice were intraperitoneally administered with 0.5 mℓ of pristane (product of Aldrich Chemical Co., Inc.). About one week later, the mice were intraperitoneally inoculated with the above-obtained hybridomas at a dose of $1 \times 10^6$ cells/mouse. About 10 days later, ascitic fluid was collected from the abdominal cavities of the mice. The fluid was centrifuged at 3,000 rpm for 10 minutes to collect a supernatant. Ammonium sulfate was added to 5 mℓ of the supernatant until the final concentration of ammonium sulfate reached 50% saturation. The resultant mixture was stirred at 4°C for 60 minutes. The mixture was then centrifuged at 10,000 rpm for 20 minutes, and the resultant sediment was dissolved in a 0.1 M tris-hydrochloric acid buffer (pH 8) and thereafter dialyzed against the same buffer. After addition of the same amount of a 1.5 M glycin-3 M sodium chloride buffer (pH 8.9), the resulting solution was subjected to chromatography on a column packed with "Protein A Sepharose CL-4B" (trade name; product of Pharmacia AB) which had been equilibrated with the same buffer.

The elution of the monoclonal antibody was conducted with a 0.1 M citrate buffer (pH 4), whereby the anti-CPBII monoclonal antibody was obtained. When CPBII-H29 was used, 22.2 mg of CPBII-A29 was obtained. 7.8 mg of CPBII-A76 from CPBII-H76, 16 mg of CPBII-A311 from CPBII-H311, and 2.9 mg of CPBII-A511 from CPBII-H511. Those anti-CPBII monoclonal antibodies exhibited the characteristics shown above in Table 1.

Example 3:

Purification of CPBII by immunoaffinity chromatography :

(1) Coupling of the anti-CPBII monoclonal antibody to carrier:

Cyanogen bromide-activated Sepharose 4B (0.4 g) was washed successively with 1 mM hydrochloric acid and a 0.1 M sodium bicarbonate-0.5 M sodium chloride buffer (pH 8.3) to prepare 1.5 mℓ of a coupling buffer of cyanogen bromide-activated Sepharose 4B.

A matching coupling buffer solution (1 mℓ) containing 2 mg of the purified monoclonal antibody CPBII-A76 was added to the former coupling buffer. The resultant mixture was shaken for 2 hours at room temperature and was then dewatered through a glass filter. Ten milliliters of a 0.1 M tris-hydrochloric acid buffer (pH 8.0) were added further and the resultant mixture was shaken for 2 hours at room temperature to block any remaining active sites.

The thus-obtained antibody-conjugated Sepharose 4B was washed three times alternately with a 0.1 M tris-hydrochloric acid-0.5 M sodium chloride buffer (pH 8.3) and a 0.1 M acetic acid-0.5 M sodium chloride buffer (pH 4.0), followed by equilibration with a 0.1 M tris-hydrochloric acid buffer (pH 7.4) to obtain an antibody column 76.

(2) Purification of CPBII by the antibody column:

The crude CPBII solution obtained in Example 1-(i)-(ii) was charged onto the antibody column 76 prepared in the above procedure. The column was washed thoroughly with the same buffer as that used for its equilibration.

The elution of CPBII can be effected (1) with a 0.1 M acetic acid-0.15 M sodium chloride buffer (pH 3.5) or (2) with a 0.2 M glycin-hydrochloric acid buffer (pH 2.3).

CPBII was not found in any through fractions. CPBII was obtained in a pure form at a recovery rate of at least 80% from eluate fractions.

Incidentally, the results of the purification are shown in terms of recovery rate in Table 2. The measurement of CPBII was conducted by a method to be described subsequently in Example 4.

Table 2

| Recovery Rate (%) of CPBII | | | |
|---|---|---|---|
| Monoclonal antibody-conjugated Sepharose | Passed fractions | Eluate fractions | |
| | | (1) | (2) |
| 76 | ND | 65 | 86 |
| Eluent (1): 0.1 M acetate-0.15 M NaCl buffer (pH 3.5). (2): 0.2 M glycin-HCl buffer (pH 2.3). In the table, "ND" indicates no detection of CPBII. | | | |

Example 4:

CPBII assay making use of anti-CPBII monoclonal antibody:

Following the procedure reported by S. Yoshitake et al. [J. Biochem, 92, 1413-1424 (1982)], horse radish peroxidase (hereinafter abbreviated as "HRP") was conjugated with the anti-CPBII monoclonal antibody. Using the resultant HRP-conjugated anti-CPBII monoclonal antibody, CPBII was measured by the ELISA method in the following manner. A solution of the monoclonal antibody in a 0.05 M sodium carbonate solution (pH 9.6) was added in 100-$\mu\ell$ portions into the individual wells of a 96-well flat-bottom microtiter plate and the walls of the wells were coated with the antibody at 25°C for 2 hours. After washing the wells with PBS-Tween, 100 $\mu\ell$ of a solution of a sample in a 0.1 M Tris-HCl-25 mM EDTA-0.05% "Tween 20" buffer (pH 7.4) was added to each of the wells. After reacting the antibody and sample overnight at 25°C, each well was washed with PBS-Tween and then added with 100 $\mu\ell$ of a diluted solution of the HRP-conjugated monoclonal antibody in PBS-Tween, followed by a reaction at 25°C for 2 hours. After washing each well with PBS-Tween, each well was added with 100 $\mu\ell$ of a substrate solution (a 0.1 M citrate-phosphate buffer containing 0.4 mg/m$\ell$ of ortho-phenylenediamine and 0.01% of hydrogen peroxide; pH 5.0), followed by a further reaction at 25°C for 30 minutes. Fifty microliters of 4.5 M sulfuric acid were added to stop the reaction and the absorbance at 492 nm was measured. As a result, CPBII in a concentration range of 1 - 100 ng/m$\ell$ has been found detectable as shown in Table 3 when CPBII-A29 is used as a coating monoclonal antibody and CPBII-A76, 311 or 511 is used as a monoclonal antibody to be conjugated or when CPBII-A511 is used as a coating monoclonal antibody and CPBII-A29 or 76 is used as a monoclonal antibody to be conjugated.

## Table 3

| Conjugated antibody / Coating antibody | A-29 | A-76 | A-311 | A-511 |
|---|---|---|---|---|
| A-29 | – | +++ | +++ | ++ |
| A-76 | + | – | ++ | + |
| A-311 | + | + | – | – |
| A-511 | +++ | +++ | – | – |

-:    inapplicable.
+,++: not successfully applicable.
+++: successfully applicable.

10

A calibration curve, which was obtained when CPBII-A29 and CPBII-A511 were used respectively as a coating monoclonal antibody and a monoclonal antibody to be conjugated, had extremely high sensitivity and moreover exhibited good linearity as illustrated in FIGURE 1.

## Claims

**Claims for the following Contracting States : DE, FR, GB, IT, NL, SE**

1. A monoclonal antibody specific to a human placenta-derived coagulation inhibitor CPBII having the following properties:

    (1) Molecular weight (SDS-polyacrylamide gel electrophoresis, reduced and non-reduced states)-:73,000±2,000;

    (2) Isoelectric point (isoelectric column electrophoresis using an ampholyte):6.2-6.6;

    (3) Stability

        (a) Inactivated by a heat treatment at 50°C for 30 minutes;

        (b) Stable in a pH range of 5.5-8.5 (at 37°C);

        (c) Stable in plasma at 37°C for 15 minutes;

    (4) Effects:

        (a) Capable of prolonging the recalcification time;

        (b) capable of prolonging the prothrombin time;

        (c) Capable of prolonging the activated partial thromboplastin time;

    (5) Analysis if amino acids:

    The existence of aspartic acid, threonine, serine, glutamic acid, proline, glycine, alanine, 1/2 cystine, valine, methionine, isoleucine, leucine, tyrosine, phenylalanine, histidine, lysine and arginine is recognized by the analysis of amino acids.

2. A hybridoma secreting a monoclonal antibody specific to a human placenta-derived coagulation inhibitor CPBII having the properties defined in Claim 1.

3. A process for purifying a human placenta-derived coagulation inhibitor CPBII, which comprises using as an immunoadsorbent a monoclonal antibody specific to the human placenta-derived coagulation inhibitor CPBII having the properties defined in Claim 1.

4. An immunoassay of a human placenta-derived coagulation inhibitor CPBII, which comprises using a conjugate of a monoclonal antibody specific to the human placenta-derived coagulation inhibitor CPBII. having the properties defined in Claim 1.

5. The monoclonal antibody in any of Claims 1 to 4, which is secreted from hybridoma CPB-II H29(Ferm BP-1753); hybridoma CPB-II H76(Ferm BP-1754); hybridoma CPB-II H311 (Ferm BP-1755) or hybridoma CPB-II H511(Ferm BP-1756).

**Claims for the following Contracting State : ES**

1. Process for preparing a hybridoma secreting a monoclonal antibody specific to a human placenta-derived coagulation inhibitor CPBII having the following properties:

    (1) Molecular weight (SDS-polyacrylamide gel electrophoresis, reduced and non-reduced states)-:73,000±2,000;

    (2) Isoelectric point (isoelectric column electrophoresis using an ampholyte):6.2-6.6;

    (3) Stability

        (a) Inactivated by a heat treatment at 50°C for 30 minutes;

        (b) Stable in a pH range of 5.5-8.5 (st 37°C);

        (c) Stable in plasma at 37°C for 15 minutes;

    (4) Effects:

        (a) Capable of prolonging the recalcification time;

        (b) Capable of prolonging the prothrombin time;

        (c) Capable of prolonging the activated partial thromboplastin time;

    (5) Analysis if amino acids:

    The existence of aspartic acid, threonine, serine, glutamic acid, proline, glycine, alanine, 1/2 cystine, valine, methionine, isoleucine, leucine, tyrosine, phenylalanine, histidine, lysine and arginine is recognized by the analysis of amino acids

which process is characterized because it comprises the following steps:

    a) obtaining antibody-secreting cells from an animal that has been immunized by using CPBII as an antigen;

    b) separately preparing myeloma cells using cell strains of an animal that belongs to the same species as that used in step (a);

    c) carrying out fusion of the cells of step (a) and the cells of step (b) in a ratio of myeloma cells to antibody-secreting cells of 1:4 to 1:10, in a suitable medium and in the presence of a fusion promoter, or else, using an electric cell fusion apparatus which relies upon high-voltage pulses;

    d) subjecting the cells from the cellular fusion of the previous step to selective growth, carrying out this step by means of diluting the cited cells in an IMDM medium followed by centrifugation, suspending the resultant sediment in a selective medium and inoculating it on a microtiter plate, after which it is cultured in a 5 % carbon dioxide culture apparatus, whereby the cells that undergo growth in this medium are hybridomas;

    e) screening the antibody-secreting hybridomas by collecting a culture with hybridomas grown therein, reacting with CPBII and then reacting the resultant product further with a second antibody which is labelled;

    f) subjecting to cloning the cells from the previous step that have been found to contain antibody-secreting hybridomas in accordance whith the limiting-dilution method, whereby a hybridoma secreting the monoclonal antibody specific to CPBII is obtained.

**2.** Process for preparing a monoclonal antibody specific to a human placenta-derived coagulation inhibitor CPBII having the properties defined in claim 1, which process is characterized in that it comprises either culturing an antibody-secreting hybridoma in a suitable culture medium and isolating the antibody of the supernatant of the culture medium; or, if desired, obtaining the monoclonal antibody on a large scale, administering peritoneally a mineral oil to an animal of the same family used to obtain the myeloma cells and then inoculating the antibody-secreting hybridoma, allowing the antibody secreting hybridoma to grow in vivo to a large volume, whereby the monoclonal antibody is formed at a high concentration in serum as well as in the ascitic fluid of the animal inoculated from which it is isolated and purified.

**3.** Process for purifying the human placenta derived coagulation inhibitor CPBII having the properties defined in claim 1, characterized in that it comprises the following steps:

    a) coupling the monoclonal antibody specific to CPBII with a solid carrier selected from dextran gel, agarose gel or polyvinyl gel;

    b) subjecting the crude CPBII to column chromatography of the carrier coupled with the monoclonal antibody as an immunoabsorbent;

    c) eluting the CPBII adsorbed on the column, whereby a highly purified CPBII is obtained.

**4.** An immunoassay process of a human placenta-derived coagulation inhibitor CPBII having the properties defined in claim 1, characterized in that it comprises the following steps:

    a) labelling amonoclonal antibody specific to CPBII, obtained by means of the processes described in the above claims, with a labelling agent selected from an enzyme, an isotope or a fluorescent substance;

    b) then adding the CPBII-containing sample to the resultant conjugate;

    c) then measuring the degree of labelling of the immunoreaction product between the CPBII and the conjugate.

**5.** Process according to any of claims 1 to 4, characterized in that the hybridoma is selected from hybridoma CPBII H29 (FERM BP-1753), CPBII H76 (FERM BP-1754), CPBII H311 (FERM BP-1755) and CPBII H511 (FERM BP-1756).

**Patentansprüche**
**Patentansprüche für folgende Vertragsstaaten : DE, FR, GB, IT, NL, SE**

**1.** Monoklonaler Antikörper, der für einen aus menschlicher Placenta gewonnenen Koagulationsinhibitor CPBII spezifisch ist, der die folgenden Eigenschaften hat:

EP 0 336 441 B1

(1) Molekulargewicht (SDS-Polyacrylamid-Gelelektrophorese, reduzierte und nichtreduzierte Zustände): 73.000 ± 2.000;
(2) isoelektrischer Punkt (isoelektrische SäulenElektrophorese unter Verwendung eines Ampholyten): 6,2 - 6,6;
(3) Stabilität
    (a) wird durch eine 30 Minuten dauernde Wärmebehandlung bei 50°C inaktiviert;
    (b) ist in einem pH-Bereich von 5,5 - 8,5 (bei 37°C) stabil;
    (c) ist in Plasma bei 37°C 15 Minuten lang stabil;
(4) Wirkungen:
    (a) kann die Rekalzifizierungszeit verlängern;
    (b) kann die Prothrombinzeit verlängern;
    (c) kann die aktivierte partielle Thromboplastinzeit verlängern;
(5) Analyse von Aminosäuren:
Durch die Analyse von Aminosäuren ist die Existenz von Asparaginsäure, Threonin, Serin, Glutaminsäure, Prolin, Glycin, Alanin, 1/2 Cystin, Valin, Methionin, Isoleucin, Leucin Thyrosin, Phenylalanin, Histidin, Lysin und Arginin bekannt.

**2.** Hybridom, das einen monoklonalen Antikörper produziert, der spezifisch ist für einen aus menschlicher Placenta gewonnenen Koagulationsinhibitor CPBII, der die in Anspruch 1 angegebenen Eigenschaften hat.

**3.** Verfahren zum Reinigen eines aus menschlicher Placenta gewonnenen Koagulationsinhibitors CPBII, umfassend den Einsatz eines monoklonalen Antikörpers als ein Immunadsorptionsmittel, der gegenüber dem aus menschlicher Placenta gewonnenen Koagulationsinhibitor CPBII spezifisch ist, der die in Anspruch 1 angegebenen Eigenschaften aufweist.

**4.** Immunoassay eines aus menschlicher Placenta gewonnenen Koagulationsinhibitors CPBII, umfassend den Einsatz eines Konjugates eines monoklonalen Antikörpers, der spezifisch ist für den aus menschlicher Placenta gewonnenen Koagulationsinhibitor CPBII, der die in Anspruch 1 angegebenen Eigenschaften aufweist.

**5.** Monoklonaler Antikörper nach einem der Ansprüche 1 bis 4, der von Hybridom CPB-II H29 (Ferm BP-1753), Hybridom CPB-II H76 (Ferm BP-1754), Hybridom CPB-II H311 (Ferm BP-1755) oder Hybridom CPB-II H511 (Ferm BP-1756) produziert wird.

## Patentansprüche für folgenden Vertragsstaat : ES

**1.** Verfahren zum Herstellen eines Hybridoms, das einen monoklonalen Antikörper produziert, der spezifisch ist für einen aus menschlicher Placenta gewonnenen Koagulationsinhibitor CPBII, der die folgenden Eigenschaften aufweist:
(1) Molekulargewicht (SDS-Polyacrylamid-Gelelektrophorese, reduzierte und nichtreduzierte Zustände): 73.000 ± 2.000;
(2) isoelektrischer Punkt (isoelektrische Säulen-Elektrophorese unter Verwendung eines Ampholyten): 6,2 - 6,6;
(3) Stabilität
    (a) wird durch eine 30 Minuten dauernde Wärmebehandlung bei 50°C inaktiviert;
    (b) ist in einem pH-Bereich von 5,5 - 8,5 (bei 37°C) stabil;
    (c) ist in Plasma bei 37°C 15 Minuten lang stabil;
(4) Wirkungen:
    (a) kann die Rekalzifizierungszeit verlängern;
    (b) kann die Prothrombinzeit verlängern;
    (c) kann die aktivierte partielle Thromboplastinzeit verlängern;
(5) Analyse von Aminosäuren:
Durch die Analyse von Aminosäuren ist die Existenz von Asparaginsäure, Threonin, Serin, Glutaminsäure, Prolin, Glycin, Alanin, 1/2 Cystin, Valin, Methionin, Isoleucin, Leucin Thyrosin, Phenylalanin, Histidin, Lysin und Arginin bekannt,
    wobei das Verfahren gekennzeichnet ist durch die folgenden Stufen:

13

a) Gewinnen von Antikörper produzierenden Zellen aus einem Tier, das unter Verwendung von CPBII als Antigen immunisiert worden ist;

b) separates Herstellen von Myelomzellen unter Einsatz von Zelllinien eines Tieres, das zur gleichen Art gehört, wie das, das in Stufe a) benutzt worden ist;

c) Verschmelzen der Zellen von Stufe a) und der Zellen von Stufe b) in einem Verhältnis von Myelomzellen zu Antikörper produzierenden Zellen von 1:4 bis 1:10 in einem geeigneten Medium und in Gegenwart eines Verschmelzungsförderers oder Verwenden einer elektrischen Zelle als Verschmelzungsvorrichtung, die mit Hochspannungs-Impulsen arbeitet;

d) selektives Züchten der Zellen aus der Zellverschmelzung der vorherigen Stufe, wobei die vorliegende Stufe ausgeführt wird durch Verdünnen der genannten Zellen in einem IMDM-Medium, gefolgt von Zentrifugieren, Suspendieren des resultierenden Sediments in einem selektiven Medium und dessen Inokulierung auf einer Mikrotiterplatte, woraufhin es in einer 5% Kohlendioxid-Kulturvorrichtung gezüchtet wird, wobei die Zellen, die in diesem Medium wachsen, Hybridome sind;

e) Screenen der Antikörper produzierenden Hybridome durch Gewinnen einer Kultur mit darin gezüchteten Hybridomen, Umsetzen mit CPBII und dann Umsetzen des resultierenden Produktes weiter mit einem markierten zweiten Antikörper;

f) Klonen der Zellen der vorhergehenden Stufe, von denen festgestellt wurde, daß sie Antikörper produzierende Hybridome enthalten, gemäß dem Grenzverdünnungs-Verfahren, wodurch ein Hybridom erhalten wird, das den gegenüber CPBII spezifischen monoklonalen Antikörper produziert.

2. Verfahren zum Herstellen eines monoklonalen Antikörpers, der spezifisch für einen aus menschlicher Placenta gewonnenen Koagulationsinhibitor CPBII ist, der die in Anspruch 1 angegebenen Eigenschaften aufweist, wobei das Verfahren dadurch gekennzeichnet ist, daß es entweder das Züchten eines Antikörper produzierenden Hybridoms in einem geeigneten Kulturmedium und das Isolieren des Antikörpers aus dem Überstehenden des Kulturmediums umfaßt oder, wenn erwünscht, Herstellen des monoklonalen Antikörpers in großem Maßstab durch peritoneales Verabreichen eines Mineralöls an ein Tier der gleichen Familie, die zum Herstellen der Myelom-Zellen benutzt wurde und Inokulieren des Antikörper produzierenden Hybridoms, Züchten des Antikörper produzierenden Hybridoms in vivo zu einem großen Volumen, wodurch der monoklonale Antikörper in einer hohen Konzentration in Serum sowie in der aszitischen Flüssigkeit des inokulierten Tieres gebildet wird, aus der er isoliert und gereinigt wird.

3. Verfahren zum Reinigen eines aus menschlicher Placenta gewonnenen Koagulationsinhibitors CPBII mit den in Anspruch 1 angegebenen Eigenschaften, dadurch gekennzeichnet, daß es die folgenden Stufen um-faßt:

a) Kuppeln des gegenüber CPBII spezifischen monoklonalen Antikörpers mit einem festen Träger, ausgewählt aus Dextrangel, Agarosegel oder Polyvinylgel;

b) Unterwerfen des rohen CPBII einer Säulenchromatographie an dem mit dem monoklonalen Antikörper gekoppelten Träger als einem Immunabsorptionsmittel;

c) Eluieren des an der Säule adsorbierten CPBII, wodurch ein hoch gereinigtes CPBII erhalten wird.

4. Immunoassay-Verfahren eines aus menschlicher Placenta gewonnenen Koagulationsinhibitors CPBII, der die in Anspruch 1 angegebenen Eigenschaften hat, dadurch gekennzeichnet, daß es die folgenden Stufen umfaßt:

a) Markieren eines gegenüber CPBII spezifischen monoklonalen Antikörpers, erhalten mittels der in den vorhergehenden Ansprüchen beschriebenen Verfahren, mit einem Markierungsmittel, ausgewählt aus einem Enzym, einem Isotop oder einer fluoreszierenden Substanz;

b) Hinzugeben der CPBII-haltigen Probe zu dem resultierenden Konjugat;

c) Messen des Markierungsgrades des Immunreaktionsproduktes zwischen dem CPBII und dem Konjugat.

5. Verfahren nach einem der Ansprüche 1 bis 4, dadurch gekennzeichnet, daß das Hybridom ausgewählt ist aus Hybridom CPBII H29, CPBII H76, CPBII H311 und CPBII H511.

EP 0 336 441 B1

**Revendications**
**Revendications pour les Etats contractants suivants : DE, FR, GB, IT, NL, SE**

1. Anticorps monoclonal spécifique d'un inhibiteur de coagulation CPBII provenant du placenta humain, ayant les propriétés suivantes :

   (1) masse moléculaire (électrophorèse sur gel de SDS et de polyacrylamide , état réduit et état non réduit) : 73 000 ± 2 000 ;

   (2) point isoélectrique (électrophorèse sur colonne isoélectrique utilisant un ampholyte) : 6,2-6,6 ;

   (3) stabilité

   (a) inactivé par un traitement thermique à 50 °C pendant 30 mm ;

   (b) stable dans un domaine de pH de 5,5 à 8,5 (à 37°C);

   (c) stable dans le plasma à 37 °c pendant 15 mm ;

   (4) effets :

   (a) capable de prolonger le temps de recalcification ;

   (b) capable de prolonger le temps de prothrombine ;

   (c) capable de prolonger le temps de thromboplastine activée ;

   (5) analyse des acides aminés :

   L'analyse des acides aminés montre l'existence de l'acide aspartique, de la thréonine, de la sérine, de l'acide glutamique, de la proline, de la glycine, de l'alanine, de la 1/2-cystine, de la valine, de la méthionine, de l'isoleucine, de la leucine, de la tyrosine, de la phényle-alanine, de l'histidine, de la lysine et de l'arginine.

2. Hybridome sécrétant un anticorps monoclonal spécifique de l'inhibiteur de coagulation CPBII provenant du placenta humain ayant les propriétés données dans la revendication 1.

3. Procédé pour purifier un inhibiteur de coagulation CPBII provenant du placenta humain, qui comprend l'utilisation comme immunoadsorbant d'un anticorps monoclonal spécifique de l'inhibiteur de coagulation CPBII provenant du placenta humain, ayant les propriétés données dans la revendication 1.

4. Analyse immunologique d'un inhibiteur de coagulation CPBII provenant du placenta humain, qui comprend l'utilisation d'un conjugué d'un anticorps monoclonal spécifique de l'inhibiteur de coagulation CPBII dérivé du placenta humain ayant les propriétés données dans la revendication 1.

5. Anticorps monoclonal selon l'une quelconque des revendications 1 à 4, qui est sécrété par l'hybridome CPB-II H29 (Ferm BP-1753) ; l'hybridome CPB-II H76 (Ferm BP-1754) ; l'hybridome CPB-II H311 (Ferm BP-1755) ou l'hybridome CPB-II H511 (Ferm BP-1756).

**Revendications pour l'Etat contractant suivant : ES**

1. Procédé pour préparer un hybridome sécrétant un anticorps monoclonal spécifique d'un inhibiteur de coagulation CPBII provenant du placenta humain, ayant les propriétés suivantes :

   (1) masse moléculaire (électrophorèse sur gel de SDS et de polyacrylamide, état réduit et état non réduit) : 73 000 ± 2 000 ;

   (2) point isoélectrique (électrophorèse sur colonne isoélectrique utilisant un ampholyte) : 6,2-6,6 ;

   (3) stabilité

   (a) inactivé par un traitement thermique à 50 °C pendant 30 mm ;

   (b) stable dans un domaine de pH de 5,5 à 8,5 (à 37°C);

   (c) stable dans le plasma à 37 °C pendant 15 mm ;

   (4) effets :

   (a) capable de prolonger le temps de recalcification ;

   (b) capable de prolonger le temps de prothrombine ;

   (c) capable de prolonger le temps de thromboplastine activée ;

   (5) analyse des acides aminés :

   L'analyse des acides aminés montre l'existence de l'acide aspartique, de la thréonine, de la sérine, de l'acide glutamique, de la proline, de la glycine, de l'alanine, de la 1/2-cystine, de la valine, de la méthionine, de l' isoleucine, de la leucine, de la tyrosine, de la phényle-alanine, de l'histidine, de la lysine et de l'arginine.

   Ce procédé étant caractérisé en ce qu'il comprend les étapes consistants :

15

a) à obtenir des cellules sécrétant un anticorps, à partir d'un animal qui a été immunisé par utilisation de CPBII comme antigène ;

b) à préparer séparément des cellules de myélome en utilisant des souches cellulaires d'un animal appartenant à la même espèce que l'animal utilisé dans l'étape (a) ;

c) à mettre en oeuvre une fusion des cellules de l'étape (a) et des cellules de l'étape (b) selon un rapport des cellules de myélome aux cellules sécrétant un anticorps de 1:4 à 1:10, dans un milieu approprié et en présence d'un promoteur de fusion, ou encore par utilisation d'un appareil électrique de fusion de cellules se fondant sur des impulsions haute tension ;

d) à soumettre les cellules provenant de la fusion cellulaire de l'étape précédente à une culture sélective, à mettre en oeuvre cette étape par dilution des cellules citées dans un milieu IMDM puis par centrifugation, à mettre en suspension le sédiment obtenu dans un milieu sélectif et à l'inoculer une plaque de microtitrage, ce après quoi on le cultive dans un appareil de culture contenant à 5 % d'anhydride carbonique, les cellules subissant la culture dans ce milieu étant des hybridomes ;

e) à sélectionner les hybridomes sécrétant un anticorps, en collectant une culture dans laquelle des hybridomes ont été cultivés, à faire réagir avec le CPBII puis à faire réagir le produit obtenu avec un deuxième anticorps marqué;

f) à soumettre à un clonage les cellules de l'étape précédente qui se sont révélées contenir des hybridomes sécrétant un anticorps par la méthode des dilutions limitantes, ce qui permet d'obtenir un hybridome sécrétant l'anticorps monoclonal spécifique du CPBII.

**2.** Procédé pour préparer un anticorps monoclonal spécifique d'un inhibiteur de coagulation CPBII provenant du placenta humain ayant les propriétés définies dans la revendication 1, ce procédé étant caractérisé en ce qu'il comprend les étapes consistant soit à cultiver un hybridome sécrétant un anticorps, dans un milieu de culture appropriée et à isoler l'anticorps du surnagent du milieu de culture ; soit, si on le souhaite, à obtenir un anticorps monoclonal à grande échelle, à administrer par voie péritonéale une huile minérale à un animal de la même famille que celle utilisée pour obtenir les cellules de myélome, puis à inoculer l'hybridome sécrétant l'anticorps, a permettre à cet hybridome sécrétant l'anticorps de croître in vivo jusqu'à un grand volume, ce par quoi l'anticorps monoclonal est formé avec une concentration élevée dans le sérum et dans le liquide ascitique de l'animal inoculé, à partir desquels il est isolé et purifié.

**3.** Procédé pour purifier un inhibiteur de coagulation CPBII provenant du placenta humain ayant les propriétés données dans la revendication 1, caractérisé en ce qu'il comprend les étapes consistant :

a) à coupler l'anticorps monoclonal specifique du CPBII avec un support solide choisi parmi le gel de dextrane, le gel d'agarose ou le gel polyvinylique ;

b) à soumettre le CPBII brut à une chromatographie sur colonne du support couplé à l'anticorps monoclonal, servant d'immunoabsorbant ;

c) à éluer le CPBII adsorbé par la colonne, ce qui donne un CPBII très purifié.

**4.** Procédé d'analyse immunologique d'un inhibiteur de coagulation CPBII provenant du placenta humain, ayant les propriétés données dans la revendication 1, caractérisé en ce qu'il comprend les étapes consistant :

a) à marquer un anticorps monoclonal specifique du CPBII obtenu par les procédés décrits dans les revendication ci-dessus, avec un agent de marquage choisi parmi une enzyme, un isotope ou une substance fluorescente ;

b) puis à ajouter au conjugué obtenu l'échantillon contenant le CPBII ;

c) puis à mesurer le degré de marquage du produit d'immunoréaction entre le CPBII et le conjugué.

**5.** Procédé selon l'une quelconque des revendications 1 à 4, caractérisé en ce que l'hybridome est choisi parmi les hybridomes CPBII H29, CPBII H76, CPBII H311 et CPBII H511.

# FIG. 1